# EUROPEAN PATENT APPLICATION

(11) **EP 3 989 233 A1**
(43) Date of publication of application: **27.04.2022**
(21) Application number: 20203092.0
(22) Date of filing: 21.10.2020
(51) Int. Cl.: G16H 10/60, G16H 20/70

(54) **PATIENT MONITORING SYSTEM**

(71) Applicant: Koninklijke Philips N.V., 5656 AG Eindhoven (NL)
(72) Inventor: BULUT, Murtaza, 5656 AE Eindhoven (NL); TIEMANN, Christian Andreas, 5656 AE Eindhoven (NL); MAESSEN, Ralph Theodorus Hubertus, 5656 AE Eindhoven (NL); BERA, Deep, 5656 AE Eindhoven (NL); VAN LIESHOUT, Ron Martinus Laurentius, 5656 AE Eindhoven (NL); SARTOR, Francesco, 5656 AE Eindhoven (NL)
(74) Representative: Philips Intellectual Property & Standards

(57) **Abstract**

A patient monitoring system comprising a memory (104) comprising instruction data representing a set of instructions (106) and a processor (102) configured to communicate with the memory and to execute the set of instructions. The set of instructions, when executed by the processor, cause the processor to obtain patient data about a patient being monitored by the patient monitoring system, obtain previous patient data, determine a difference between the patient data and the previous patient data; and send a message to a user display associated with the patient monitoring system, to cause the user display to adapt content displayed on the user display, based on the determined difference.

## Description

### FIELD OF THE INVENTION

The disclosure herein relates to patient monitoring systems and methods performed by patient monitoring systems.

### BACKGROUND OF THE INVENTION

Patient monitoring systems such as clinical decision support systems (CDSS) are used in medical facilities (e.g. hospitals and/or clinics) to monitor patients by reporting patient vital signs, treatments given and/or any other patient information to the clinical user (doctor, nurse, clinician etc) responsible for the patient's care. There is a need for simple yet informative interfaces for patient monitoring systems and generally for solutions that generate patient related information, so that the information can be quickly processed and reliably acted upon. One challenge is that different clinical users find different information more useful than others and therefore the definition of "simple" and "informative" are highly personal and depend on the clinical user using the patient monitoring system. Thus patient monitors and CDSSs have been developed that can be adapted according to the preferences of the clinical user caring for the patient.

Feedback from clinical users suggests that current advanced monitoring solutions may not be being used to their full capacity. For example, some clinical users report that the screens with detailed and complicated outputs are often not regularly used. Functionality for creating patient-specific profiles may also be overlooked.

It is an object of embodiments herein to provide improved patient monitoring systems to aid clinical users to make improved clinical decisions.

### SUMMARY OF THE INVENTION

Research shows that many of the poor decisions made by clinical users can be linked to a phenomena known as patient similarity perception (e.g. where clinicians assume that a current patient having similar pathology to a previous patient, will progress in the same manner as the previous patient) and the behavior originating from this. For example, the paper by Khairat S, Marc, D., Crosby W., Sanousi A.A., entitled "Reasons for physicians not adopting clinical decision support systems: critical analysis", JMIR Med Inform, vol. 6, iss. 2, 2018 notes that *"Most clinicians exhibit bias when it comes to medical information that they know and, therefore, they typically focus on things that would agree with the specific clinical outcome that they want to see in their patients".*

Common cognitive errors in clinical decision making include, "Anchoring" whereby a clinical user relies on initial impressions too early in the diagnostic process and thus fails to adjust their initial impressions in light of new information, "Availability" whereby a clinical user judges a situation as more likely or frequent if it easily comes to mind, and "Framing bias" whereby a clinical user tends to be swayed by subtleties in how a situation is presented (e.g., description of the risks and benefits of treatment options). These are explained in more detail in the consensus study report entitled "Best Care at Lower Cost: The Path to Continuously Learning Health Care in America", Mark Smith, Robert Saunders, Leigh Stuckhardt, and J. Michael McGinnis, Editors. Embodiments herein aim to help a clinical user to reduce clinical errors of these types.

According to a first aspect, there is provided a patient monitoring system comprising a memory comprising instruction data representing a set of instructions, and a processor configured to communicate with the memory and to execute the set of instructions. The set of instructions, when executed by the processor, cause the processor to: obtain patient data about a patient being monitored by the patient monitoring system, obtain previous patient data, determine a difference between the patient data and the previous patient data, and send a message to a user display associated with the patient monitoring system, to cause the user display to adapt content displayed on the user display, based on the determined difference.

There is thus provided improved clinical decision support systems and patient monitoring systems that highlight differences between the data of the patient being monitored by the patient monitoring system and previous patient data (either intra-patient differences or inter-patient differences). By highlighting these differences, the clinical user is encouraged to consider how the current patient is different to what has gone before, thereby reducing some of the types of clinical bias described above (e.g. anchoring, availability and framing bias). Thus the clinical user (e.g. medical professional) is aided to make an unbiased clinical decision. The output of the system can further be used as an alert that warns the medical professional about the discrepancy so as to reduce any biased decision making.

Such measured differences may trigger an alarm to the medical professional when they make a decision - to remind them that this patient is different e.g to previously examined patients. In this way, the clinical users may be encouraged to pay more attention to the differences exhibited by the current patient, thereby reaching to an optimum clinical decision which is less impacted by previous experience learned from previous patients.

Since a better decision may be reached more quickly, embodiments of the disclosure herein improve patient outcomes, which may be reflected in shorter hospital stays and fewer interventions and/or medications. This can contribute to better use of hospitals' physical (lab space, equipment, bed) and personal (nurse, technicians, physicians) resources, which further contributes to lowering the monetary cost of the patient care.

In summary, the disclosure herein provides improved clinical decision support for diagnosis and treatment of patients.

According to a second aspect there is a method performed by a patient monitoring system, the method comprises: obtaining patient data about a patient being monitored by the patient monitoring system, obtaining previous patient data, determining a difference between the patient data and the previous patient data, and sending a message to a user display associated with the patient monitoring system, to cause the user display to adapt content displayed on the user display, based on the determined difference.

According to a third aspect there is a computer program product comprising a computer readable medium, the computer readable medium having computer readable code embodied therein, the computer readable code being configured such that, on execution by a suitable computer or processor, the computer or processor is caused to perform the method of the second aspect.

These and other aspects will be apparent from and elucidated with reference to the embodiment(s) described hereinafter.

### BRIEF DESCRIPTION OF THE DRAWINGS

Example embodiments will now be described, by way of example only, with reference to the following drawings, in which:
Fig. 1 shows an example patient monitoring system according to embodiments herein;
Fig. 2 shows an example method according to embodiments herein;
Fig. 3 shows an example method according to some embodiments herein;
Fig. 4 shows an example method according to some embodiments herein; and
Fig. 5 shows an example method according to some embodiments herein.

### DETAILED DESCRIPTION OF EMBODIMENTS

As described above, clinical users (e.g. doctors, clinicians, nurses, researchers etc) may exhibit subconscious bias in their decision making, for example, "anchoring", "availability" and/or "framing" bias. The discussion of clinical decision errors above shows that many of the errors are not due to lack of clinical knowledge, but due to subjective (psychological) factors, such as relying too much (without consciously realizing it) on assumed or first available knowledge.

Patient monitors such as CDSS help clinical users to make clinical decisions about their patients. Some monitors have different profiles stored. The profiles are generally patient based. For example, one profile can be for an adult patient, and another for a 5-year old child. By switching from one profile to another, measurement settings (e.g. alarm limits) and display settings can be changed.

Information displayed on patient monitors is often pre-set, meaning that the signal types, graphics, calculated or estimated parameter types, and update rates are predetermined (e.g. for each profile type, where applicable). Some systems also allow clinicians to make changes to some pre-set settings. In practice however, such adaptations may be difficult to do due to lack of time, or not knowing what changes are possible, or simply due to lack of interest or perceived need to make changes.

The disclosure herein aims to provide improved methods and systems for patient monitoring. In particular by providing timely and relevant information to the clinical user of the monitor to aid the clinical user to make better decisions and reduce clinician-perception linked clinical errors.

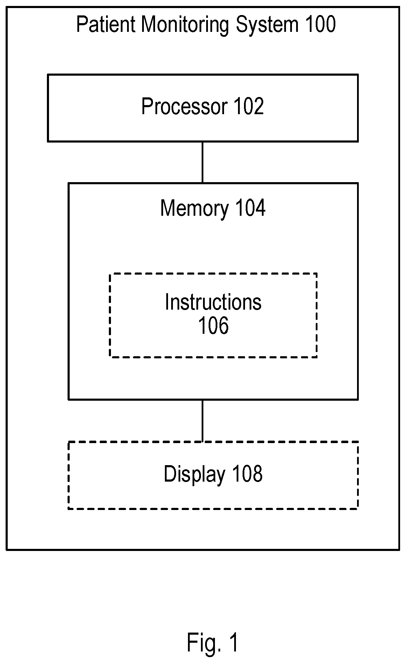

Turning now to Fig. 1 in some embodiments there is a patient monitoring system 100 for use in monitoring a patient, according to some embodiments herein. Generally, the patient monitoring system may form part of a computer apparatus or system. The patient monitoring system 100 may be comprised in a monitor such as a bedside monitor, or CDSS. Alternatively, the patient monitoring system may be comprised in a laptop, desktop computer or other computing device. In some embodiments, the patient monitoring system 100 may form part of a distributed computing arrangement or the cloud.

Alternatively, the patient monitoring system 100 may be comprised in a pager, or any other device (not necessarily comprising an interactive display) e.g. that is capable of generating alerts. Such alerts can be visual, haptic, auditory, etc. For instance, a pager may generate a signal to alert a clinical user (e.g. when examining a patient), to indicate to the clinical user that this patient is different to previous patients.

The patient monitoring system comprises a memory 104 comprising instruction data representing a set of instructions and a processor 102 (e.g. processing circuitry or logic) configured to communicate with the memory and to execute the set of instructions. Generally, the set of instructions, when executed by the processor, may cause the processor to perform any of the embodiments of the method 200 as described below.

More specifically, the set of instructions, when executed by the processor, cause the processor to: obtain patient data about a patient being monitored by the patient monitoring system, obtain previous patient data, determine a difference between the patient data and the previous patient data, and send a message to a user display associated with the patient monitoring system, to cause the user display to adapt content displayed on the user display, based on the determined difference.

The processor 102 can comprise one or more processors, processing units, multi-core processors or modules that are configured or programmed to control the patient monitoring system 100 in the manner described herein. In particular implementations, the processor 102 can comprise a plurality of software and/or hardware modules that are each configured to perform, or are for performing, individual or multiple steps of the method described herein. The processor 102 can comprise one or more processors, processing units, multi-core processors and/or modules that are configured or programmed to control the patient monitoring system 100 in the manner described herein. In some implementations, for example, the processor 102 may comprise a plurality of (for example, interoperated) processors, processing units, multi-core processors and/or modules configured for distributed processing. It will be appreciated by a person skilled in the art that such processors, processing units, multi-core processors and/or modules may be located in different locations and may perform different steps and/or different parts of a single step of the method described herein.

The memory 104 is configured to store program code that can be executed by the processor 102 to perform the method described herein. Alternatively or in addition, one or more memories 104 may be external to (i.e. separate to or remote from) the patient monitoring system 100. For example, one or more memories 104 may be part of another device. Memory 104 can be used to store the patient data, the previous patient data, the determined difference and/or any other information or data received, calculated or determined by the processor 102 of the patient monitoring system 100 or from any interfaces, memories or devices that are external to the patient monitoring system 100. The processor 102 may be configured to control the memory 104 to store the patient data, the previous patient data, the determined difference, and any other information or data received, calculated or determined by the processor.

In some embodiments, the memory 104 may comprise a plurality of sub-memories, each sub-memory being capable of storing a piece of instruction data. For example, at least one sub-memory may store instruction data representing at least one instruction of the set of instructions, while at least one other sub-memory may store instruction data representing at least one other instruction of the set of instructions.

It will be appreciated that Fig. 1 only shows the components required to illustrate this aspect of the disclosure and, in a practical implementation, the patient monitoring system 100 may comprise additional components to those shown. For example, the patient monitoring system 100 may further comprise the user display. A user display may comprise, for example, a screen, a computer screen, and/or a screen on a mobile phone or tablet. The patient monitoring system may comprise a stand or trolley, for example, as part of a bedside patient monitoring system. The patient monitoring system may further comprise a user input device, such as a keyboard, mouse or other input device that enables a user to interact with the patient monitoring system, for example, to provide initial input parameters to be used in the method described herein. The patient monitoring system 100 may comprise a battery or other power supply for powering the patient monitoring system 100 or means for connecting the patient monitoring system 100 to a mains power supply.

In some embodiments, the patient monitoring system may further comprise one or more sensors or monitors for obtaining the patient data. Alternatively or additionally, one or more sensors may be external to the patient monitoring system 100 and the patient monitoring system may be adapted to receive data from such monitors, e.g. via a wired or wireless connection.

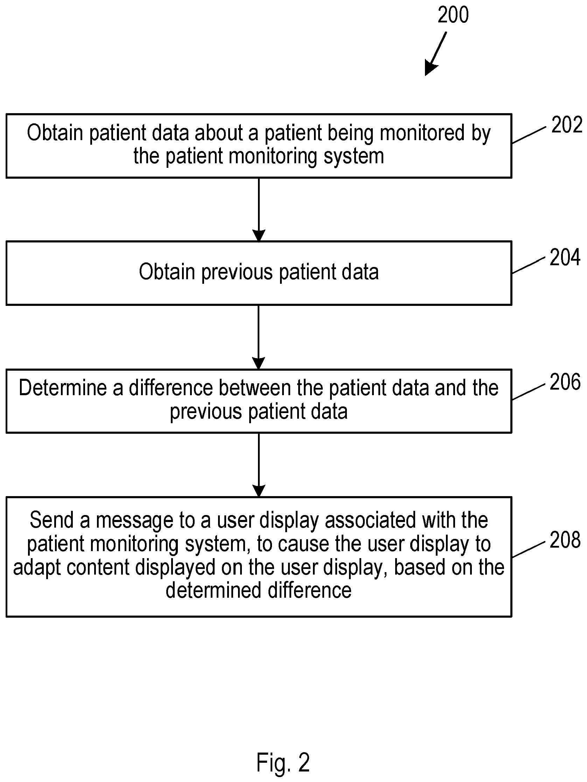

Turning now to Fig. 2, there is a computer implemented method 200 performed by a patient monitoring system. Embodiments of the method 200 may be performed, for example by a patient monitoring system such as the patient monitoring system 100 described above.

Briefly, in a first step 202, the method 200 comprises obtaining patient data about a patient being monitored by the patient monitoring system. In a second step 204 the method comprises obtaining previous patient data. In a third step 206 the method comprises determining a difference between the patient data and the previous patient data. In a fourth 208 step the method comprises sending a message to a user display associated with the patient monitoring system, to cause the user display to adapt content displayed on the user display, based on the determined difference.

In this manner, a patient monitoring system may be provided that is capable of adapting content displayed on the user display, based on the determined difference between the patient data and the previous patient data. Thus, patient differences are emphasised in a manner that ensures that the clinical user is aware of them. This may be used to indicate to the clinical user how the patient data has changed (inter-patient differences) or how the patient is different to a previous patient, or previous patient cohort (intra-patient differences). In this way, the clinical user is encouraged to consider the differences between the patient data and what has gone before and is thus less likely to make decisions made on the basis of bias. The technical effect is thus a patient monitor configured to provide content displayed on a user display in a manner that discourages bias in a human operator and thus contributes to reducing or eliminating clinical errors.

In more detail, in step 202, the patient data is obtained about a patient being monitored by the patient monitoring system (also referred to herein as "the patient", or "the current patient"). The patient being monitored may be connected to one or more sensors or monitors, for example, a heart rate monitor, a blood oxygen saturation monitor (e.g. an SpO2 sensor), or any other type of sensor. The patient data may comprise sensor readings, or other data from such monitors.

The patient data may alternatively or additionally comprise information about the patient's (e.g. the patient being monitored's) medical history, physiological measurements, treatment plans, or diagnostic data. The patient data may indicate a treatment protocol being followed for the patient, and/or any deviations from said treatment protocol. The patient data may further indicate the results of any lab tests performed on the patient being monitored.

The patient data may alternatively or additionally comprise information about the patient being monitored's medical history, age, sex, ethnicity, and/or any other socioeconomic data about the patient.

The patient data may alternatively or additionally comprise any data derivable from any of the types of patient data listed above. For example, data generated by analysing or processing existing patient data, e.g. by means of a computational resource such as a machine learning model, or other mathematical algorithm.

The patient data may be obtained, for example from a patient database (e.g. such as the patient's medical history), or one or more sensors or pieces of monitoring equipment. In some embodiments, the patient data is obtained in real time to reflect the patient's current situation.

With respect to the previous patient data, as discussed in more detail below, the previous patient data may comprise intra-patient data, for example, data about other (e.g. previous) patients. Alternatively or additionally, the previous patient data may comprise inter-patient data (for example earlier or historical data about the patient being monitored).

The previous patient data may comprise data of the same type as the patient data, e.g. the same type of sensor readings, the same socioeconomic data etc. As such the previous patient data may be comparable (e.g. in format or content) to the patient data.

The previous patient data may be obtained from a database, for example, a database of medical records, or a database of sensor readings.

In some embodiments, the method further comprises selecting one or more previous patients from a plurality of previous patients, and obtaining the previous patient data about the selected one or more previous patients. For example, the previous patients may be selected from a database of previous patients.

In some embodiments, the method comprises determining the current clinical user of the clinical monitoring system, and selecting one or more previous patients that were treated by i) the current clinical user, ii) a team of clinicians associated with the current clinical user, or iii) a medical facility associated with the current clinical user. In this way, previous patients/cases that are likely to be familiar to the current user may be selected for the comparison. The clinical user will be familiar with these past cases, and potentially will use learnings from them for the diagnosis and treatment of the current patient. This enables the clinical user to better appreciate the differences between the current patient and their previous patients, and thus reduce biased decision making.

In such embodiments, the identity of the clinical user may be obtained, for example, by any method suitable for identifying an individual. Examples include, but are not limited to, identification (ID) scanning, device log-ins, shift (working) times, location tracking, fingerprint scanning, and iris scanning. For example, in some embodiments of the patient monitor 100 described above, the patient monitor may further comprise a scanner for scanning an ID card or badge, a user log-in, a fingerprint scanner, an iris scanner and/or any other hardware suitable for identifying a clinical user.

In some embodiments, alternatively or additionally, selecting one or more previous patients from a plurality of previous patients may comprise selecting one or more previous patients that have at least one characteristic in common with the patient. Examples of characteristics include, but are not limited to, at least one physical characteristic in common with the patient, at least one symptom in common with the patient, at least one treatment received in common with the patient (e.g. a common treatment protocol followed, common deviation from a treatment protocol or a lab test performed in common) and/or at least one treatment outcome in common with the patient.

In some embodiments, the method may comprise selecting one or more previous patients having a similarity metric that satisfies a predetermined criteria. For example, the predetermined criteria may comprise the n most similar patients. In other words, the most similar patient or patients.

The purpose of selecting similar patients is to find individual, or groups of previous patients who are the closest to the current patient, so that the differences with respect to the current patient can be highlighted. These differences may represent the most pertinent information that should be considered when diagnosing the current patient, and may thus make the difference between making an accurate diagnosis for the current patient, compared with making a biased diagnosis based on the previous patient(s). When selecting similar patients for comparison to the current patient, an option could be to take treatment outcomes (if available) into account as well, e.g., complications, length of stay, readmission, survival. Depending on the situation one may be interested in similar patients for which the treatment was successful (or perhaps was not successful). This may help to prevent poor decision-making and past mistakes from being repeated in the current situation.

Similar patients may be selected based on a clustering procedure. For example, in some embodiments, the method may comprise performing a clustering process on the patient data and data of the plurality of previous patients, and selecting the one or more previous patients from a cluster comprising (e.g. associated with or including) the patient being monitored by the patient monitoring system. In other words, selecting the one or more previous patients based on a cluster overlapping with, or having the most features in common with the patient being monitored. The skilled person will be familiar with different methods of clustering patients. For example, k-means clustering.

In some embodiments, clinician-patient (data) interaction characteristics may be used to cluster patients. Not every patient in the group of patients treated by a clinical user will be familiar to the clinical user in the same manner or to the same extent. If for example, the clinical user has spent more time with some patients than others, these will be more familiar to him/her. So these aspects may also be taken into account while determining the clusters, or while computing the difference.

Some example clinician - patient (data) interaction aspects which may be considered are: time spent with the clinical user, notes, feedback, health records generated by the clinical user, team size and compositions, role of the clinical user in the team, protocols followed, examinations performed, standard operating procedures, and/or lab tests performed by the clinician. Such adaptations are useful for further personalizing the difference calculations according to the clinician data.

In some embodiments, the method comprises re-performing the clustering process upon a change in the patient data and sending a second message to the user display to cause the user display to provide a notification if the patient moves into a different cluster. For example the re-clustering may be performed periodically or in response to a change in the patient data. By monitoring changes in the current patient compared to clusters of previous patients, new symptoms consistent with new pathologies may be determined. The clustering process may also be re-performed in response to changes in the previous patients e.g. the group of patients with which the current patient is being compared against. Similar to the current patient changing, the condition of the previously treated patients may also change.

In some embodiments, the one or more previous patients may be selected from the plurality of previous patients such that the determined difference between the selected one or more patients and the patient satisfies a predefined criteria. For example, a cohort of previous patients (or reference patients) may be selected so as to find patients with (e.g. according to) a threshold difference level.

In embodiments where the previous patients are selected from those having been treated previously by the clinical user, clinical user's team or hospital (inter-patient differences), and in scenarios where there are too few previous patients from which to select one or more patients for which the difference satisfies the predefined criteria, then the plurality of previous patients may be supplemented with patients from a database of patients. Such a database may comprise a stock or standard database. Such a database may comprise patients e.g that were treated by different clinicians and/or different teams of clinicians and/or treated at different hospitals.

Including patient records from other hospitals, or patients not treated by the clinician or the care team may be beneficial when the pool of patients to compare the current patient against is not big or diverse enough. In order to make the clinician aware of the other feasible options, the patient pool can be expanded based on a predefined criteria that the difference must satisfy. For example, if the differences are not big enough, or many enough then the pool of patients can be extended by including similar types (e.g. cardiac, age >40) of patients from other hospitals.

An example embodiment describing such a process is described in the following four step process:
Step 1: Select (e.g. by the clinician, or automatically as a function of the patient condition, such as more uncertain patients are evaluated against a larger pool of patients) the difference profile that is going to be used. As an example, a predefined criteria could be as follows:
   Root mean square value difference between signal 1 (patient data) and signal2 (previous patient data) > threshold 1
   Number of previous patient vitals that satisfy above criteria > threshold2
   Number of hospitals where previous patients were treated > threshold3
Step 2: Compute the differences selected in the previous step, if criteria satisfied stop, otherwise continue.
Step 3: Include other previous patients, according to a defined similarity criteria, which can be linked or is part of the difference profile selected above.
Step 4: Go to step 2, and continue until the predefined criteria is satisfied.

In this way, it is possible to change the group size of previous patients that are compared to the current patient being monitored, until a difference satisfying the predefined criteria is found. This can prevent tunnel vision by e.g. increasing the patient pool until, say n differences are found. The technical effect of this is that even low probability differences (and thus low probability conditions) may be detected and highlighted to the clinical user in this manner.

Furthermore, the patient history information, e.g. whether the patient is a cardiac surgery patient or trauma, can be also used for filtering. This can be done either before, or after the signal-based patient clustering described above.

In addition, information on how patients were treated, including the protocols followed, deviations from the protocol, time of treatment, size and expertise of the team involved in the treatment can be used for grouping the patients, for example, taking into account the amount of time the clinical user spent on looking at the SOP/Guidelines and the amount of time the clinical user deviated from the SOP/Guidelines. The frequency and the deviation from the SOP/Guidelines will tell the system how familiar the clinical user is with this type of patient. If the frequency is low and the deviation is small the clinical user has a high degree of accuracy in treating this patient correctly. If the deviations are high, this may indicate that the clinician was inexperienced, or that the patient case was difficult.

Turning now to step 206, once the previous patient data is obtained, then the method comprises determining a difference between the patient data and the previous patient data.

Three example ways of determining a difference are as follows.

Difference computation example 1: signal to signal, or data to data differences: Here comparable (e.g. of the same type) signal or data are compared and their differences are reported. For example, a distribution of the patient data can be compared to the distribution of the previous patient data. For example, the patient data can be processed to select or calculate features, for which the distributions can be compared. Example features, include features related to patient physiological signals such as heart rate, respiration rate, blood pressure and so on, medication pharmacodynamics and pharmacokinetics, response to treatment, genomics related features. As indicated, these features can be measured features, such as blood pressure, or features calculated from the patient data, such as pressure at a selected part of aorta, or likelihood of internal bleeding. Various metrics exist to analyse the difference between distributions, e.g., the Kullback-Leibler divergence, Hellinger distance, various entropy metrics, and distance metrics.

Difference computation 2: signal or data interpretation differences: Here the patient data (e.g. sensor signals and/or other raw data) is first processed and analyzed (for example using a machine learning algorithm), and the processed outputs are compared (instead of the raw data). Processed outputs include, for example, a diagnosis as predicted by a machine learning algorithm.

As another example, the distribution of the patient data can be used to determine/predict a potential list of treatment options, and this list can be compared against a list of treatment options estimated from processing of the previous patient data. As another example, vital signs may be used to determine several scores such as hemodynamic stability, APACHE, SAPS and the difference between these scores can be used in step 206. Example methods that can be used for these calculations include similarity distances, such as Cosine distance, correlation, and intersection measures.

Difference computation 3: Combination of computation 1 and computation 2: Here the output can include both metrics calculated directly from the raw data, and from the processed data. For example, the list of options in the computation 2 can be accompanied by statistics showing the amount of the patient data.

It will be appreciated that these are only examples however, and that a wide range of differences between the patient data and the previous patient data may be determined.

The examples above describe methods to compute the difference between two (processed) signals, while a patient is characterized by multiple signals and sources of information. Several options are possible to quantify the overall difference between two patients. For example, the average or maximal difference of all signal differences can be calculated. Furthermore, many metrics like the Kullback-Leibler divergence can be extended to multivariate probability distributions.

The different signals (e.g. different types of sensor data, or processed data) may have different scales. If so, this may be taken into account when calculating an overall difference between two patients. Normalization of the signals may be performed, for example, to prevent one signal dominating the overall difference score. One could also apply different weighing factors to the signals, e.g., to increase the contribution of a signal when it is considered to be more important than others.

In the computation of the difference, data from the current patient (patient1), let us call this signal1, is compared to the data of previous patients (group1), let us call this signal2. Signal2 can be determined in several ways. Two example methods are provided as follows:
Method 1: Each patient in group 1 can be treated individually: For example data from patient1 can be compared to relevant data of individual patients in group 1, the patient (patient2) with the most similar data can be selected from group1, and the signal2 can be selected from the signals belonging to the patient2. Instead of a single patient, multiple patients from group1 can be also selected, and the same steps repeated for each patient.
Method 2: Clustering patients in group1: Patients in group1 can be clustered, for example based on the their similarity (e.g. symptoms, type, SOP/guidelines, treatment time, team size, amount and type of patient data, etc.), and then a cluster (cluster1) with the most similar characteristics to patient1 can be selected, and the signal2 can be determined from the cluster1 patient data (e.g. by taking the average, or selecting one, or generating a signal, etc.).

It will be appreciated that the methods above are examples only and that other methods are also possible.

Performing pairwise comparisons between patients like in method 1 may become challenging when the total set of signals is not identical between patients (e.g., patient 1 has signals A, B, C, patient 2 has signals A and B, and patient 3 has signals B and C). Ranking patients based on a similarity metric may become difficult in this case, although there are options to deal with this. For example, one could use the intersection of all available signals, or use the maximal/average identified signal difference. This challenge is less likely to occur with method 2, because a patient is compared to a cluster average for which likely a rich set of signals is available.

Once a difference is determined, the method moves to step 208 comprising: sending a message to a user display associated with the patient monitoring system, to cause the user display to adapt content displayed on the user display, based on the determined difference.

Generally, in embodiments above wherein the previous data relates to previous patients other than the current patient being monitored (and thus inter-patient differences are determined in step 206), then the message may cause the user display to highlight (e.g. indicate or draw attention to), to the current clinical user, the difference between the patient and the one or more previous patients.

In embodiments above wherein the patient data comprises current data about the patient being monitored by the patient monitoring system and the previous patient data comprises previous data about the patient being monitored by the patient monitoring system (and thus intra-patient differences are determined in step 206), the method may further comprise sending the message to the user display to cause the user display to indicate (e.g. highlight or draw attention to) differences between the current data and the previous data of the patient.

Generally, the message may instruct or cause the user display to order (or reorder) content displayed on the display so as to highlight the difference to the user. For example, signals having the greatest difference may be put to the top of the user display (e.g. top of the monitor screen) or in a position most likely to be viewed first by the clinical user. The content may be ordered according to the difference measured between each signal type.

In one example, the message may instruct or cause the user display to state or highlight the differences. In another example, the message may instruct or cause the user display to suggest (or give the option) to the clinical user that they change the settings of the patient monitor. For example, by providing an option to select or generate a new patient profile, alarm settings, display settings, sensor settings, etc. (for the patient monitor).

Other ways that the differences may be highlighted/indicated to the user include but are not limited to:
- A signal typically not displayed on the main screen can be (calculated and) displayed, or the order of signals altered, or some signals graphically emphasized (e.g. by coloured symbols, outlining etc)
- The user display settings can be adjusted to display one signal instead of the other (e.g. display pressure signal instead of respiration signal)
- The differences in the treatment protocols can be emphasized
- The differences in (patient) data collection methods, or data size can be emphasized
- The context differences can be emphasized
- The order with which the processing results are displayed or presented can be determined based on the calculated differences. First display the information that is the most different, and only later display similar information.
- Timing of the information (e.g. output of the difference module) presentation (e.g. notification to the clinician) can be adjusted to be during the periods (or patient states) where the differences are observed or expected to be the greatest. For example, if it is expected that during a time interval1 (e.g. after intake of a medication1, or specific user activity), the differences between the patient data (signal1) and the previous patient data (signal2) will be the greatest, then the notification to the clinician can be generated during this period.
- The notification/trigger threshold can be altered (Note that the alarm threshold may be kept the same to make sure that any life-threatening situation is still noticed).

As described above, one benefit of this step is that it can potentially make/force the clinical user of the monitor (doctor, nurse etc) to notice the differences between the current patient and the other patients that they have treated or are currently treating. Thus reducing clinical bias errors. There is thus provided an improved and dynamic patient monitoring system with improved signal processing and interpretation capabilities that tailors displayed content to the current patient/clinical user combination in a manner that helps reduce clinical bias errors of the clinical user.

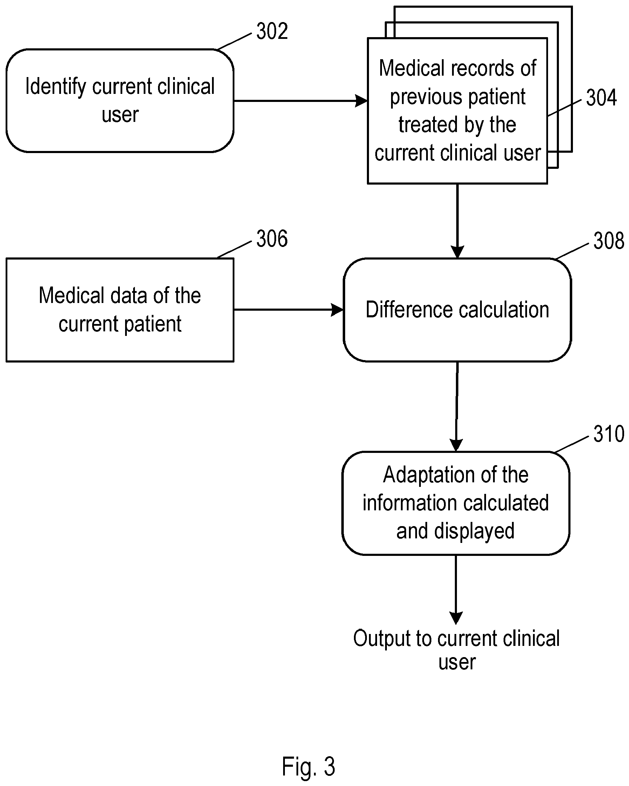

Fig. 3 illustrates an embodiment of the method 200. In this embodiment, the current clinical user is identified in step 302. Patient data about the patient being monitored by the patient monitoring system is obtained in step 306. Previous patient data from previous patients treated by the identified current clinical user is obtained in 304 (for example from medical records). A difference between the patient data and the previous patient data is determined in step 308 and a message is sent to a user display associated with the patient monitoring system in step 310 to cause the user display to adapt content displayed on the user display, based on the determined difference.

In this way, physician specific inter- and intra-patient differences may be calculated, and the generated output adapted, taking the differences into account. Thus an intelligent patient monitor is provided that can help a clinical user to reduce bias when diagnosing and treating a patient.

The difference is determined according to any of the measures described above with respect to step 206 and the detail therein will be understood to apply equally to step 306. Adaption of the content displayed on the user display was described in detail above with respect to step 308 and the detail therein will also be understood to apply equally to the step 310.

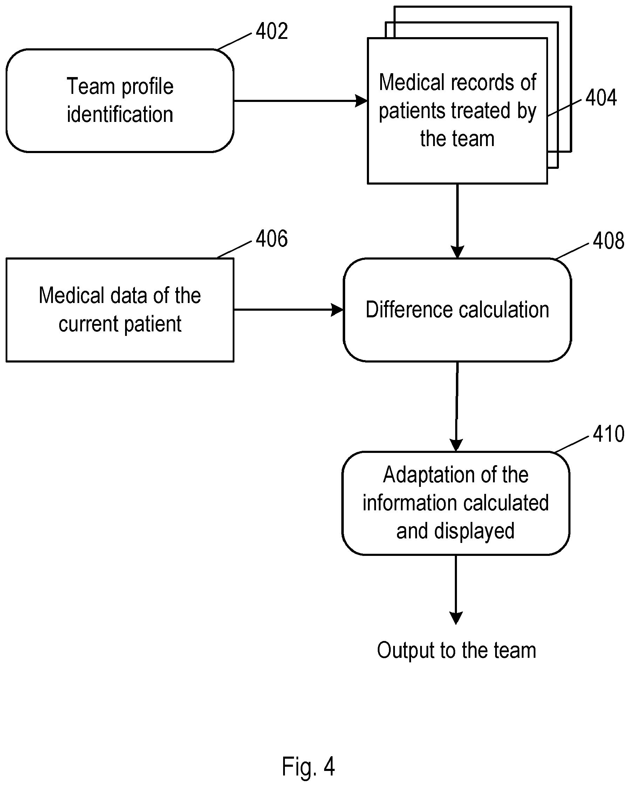

Turning now to Fig. 4, which shows another example embodiment. In this embodiment, instead of using only the physician profile, the profile of the team treating the patient can be used. In this case, first the people in the team with decision power (e.g. physician, resident, senior nurse) are determined. The patient previously treated by these selected team members are included in the group of other patients. This is based on the insight that clinical users operate in teams and therefore the expertise of the clinical user is a product of the expertise of the whole team. The clinical user's team consists of different persons with different expertise and responsibilities. By combining and comparing the patient groups associated with the different caregivers, a better difference calculation can be made.

Thus in Fig. 4, a team of clinicians associated with the current clinical user is identified in step 402 (e.g. a team profile is identified). Patient data about the patient being monitored by the patient monitoring system is obtained in step 406. Previous patient data from previous patients treated by the identified team of physicians is obtained in step 404 (for example from medical records). A difference between the patient data and the previous patient data is determined in step 408 and a message is sent to a user display associated with the patient monitoring system in step 410 to cause the user display to adapt content displayed on the user display, based on the determined difference.

The difference is determined according to any of the measures described above with respect to step 206 and the detail therein will be understood to apply equally to step 408. Adaption of the content displayed on the user display was described in detail above with respect to step 208 and the detail therein will also be understood to apply equally to the step 410.

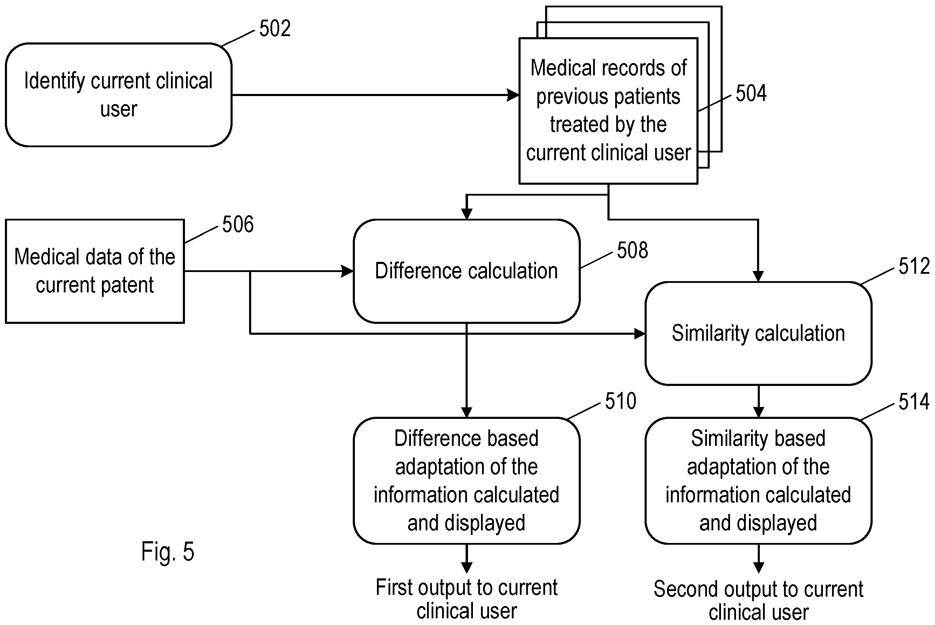

Turning now to Fig. 5, which shows another example embodiment. In this embodiment, in addition to using (patient) difference information, (patient) similarity information can be also used. For example, the content on the user display may first be adapted as a function of the differences, and once examination of these has been acknowledged by the clinical user, the patient data as a function of patient similarity may then be displayed to the user. By displaying first the difference information, the decision making bias (explained in the introduction) can be minimized. Generally, therefore, in this embodiment, the differences may be presented before (e.g. no later than) the similarities, which means that they may be presented earlier or simultaneously.

Thus in Fig. 5, the current clinical user is identified in step 502. Patient data about the patient being monitored by the patient monitoring system is obtained in step 506. Previous patient data from previous patients treated by the current clinical user is obtained in step 504 (for example from medical records). A difference between the patient data and the previous patient data is determined in step 508 and a message is sent to a user display associated with the patient monitoring system in step 510 to cause the user display to adapt content displayed on the user display, based on the determined difference. In step 312, a similarity is determined and, subsequent to the content on the display being adapted according to the difference, the content on the user display is further adapted 314 according to the similarity.

The difference is determined according to any of the measures described above with respect to step 206 and the detail therein will be understood to apply equally to step 508. Adaption of the content displayed on the user display was described in detail above with respect to step 208 and the detail therein will also be understood to apply equally to the step 510.

The skilled person will be familiar with ways in which similarity can be calculated. For example, through the use of cosine distance, correlation, intersection, and clustering methods. Examples of similarities may include similarity in complexity, length of stay, change of complication development, clinical outcome, etc. It is noted that the same or different data may be used when determining the similarities and differences. Furthermore, the means of presenting the similarities can be different (or the same) to the means of presenting the differences.

Turning now to another embodiment, as described above, in some embodiments, the method may comprise performing a clustering process on the patient and the plurality of previous patients, and selecting the one or more previous patients from a cluster comprising (e.g. associated with or including) the patient being monitored by the patient monitoring system. The method may further comprise re-performing the clustering process upon a change in the patient data and sending a second message to the user display to cause the user display to provide a notification if the patient moves into a different cluster.

The speed of change may also be determined. For example, if a patient quickly moves from one cluster to another, this may be highlighted to the clinical user of the patient monitoring system.

In this way, the difference and/or similarity calculation is done at the intake (for example, as a part of the patient intake, difference calculation profile is selected) of the patient being monitored to determine the most optimal information transfer towards the caregiver. However, when the patient situation changes and the patient 'drifts' towards another cluster (or "patient similarity group") the system can notify the caregiver of an adaptation of the optimal information transfer based on the new situation of the patient.

The 'drift' of the patient from the primary selected cluster can also be used as weighing factor for the 'adaptation of the information calculated and displayed', for example when the patients Kullback-Leibler divergence value changes the initial classification is not correct. The rate of change can also trigger different events. When there is a fast change the patient is either incorrectly classified (i.e. large error) or the patient is deteriorating. In those case the 'adaptation of the information calculated and displayed' should be reduced. When the change is slow a new classification is needed.

Turning now to other embodiments, in another embodiment, there is provided a computer program product comprising a computer readable medium, the computer readable medium having computer readable code embodied therein, the computer readable code being configured such that, on execution by a suitable computer or processor, the computer or processor is caused to perform the method or methods described herein.

Thus, it will be appreciated that the disclosure also applies to computer programs, particularly computer programs on or in a carrier, adapted to put embodiments into practice. The program may be in the form of a source code, an object code, a code intermediate source and an object code such as in a partially compiled form, or in any other form suitable for use in the implementation of the method according to the embodiments described herein.

It will also be appreciated that such a program may have many different architectural designs. For example, a program code implementing the functionality of the method or system may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A patient monitoring system comprising:
a memory (104) comprising instruction data representing a set of instructions (106); and
a processor (102) configured to communicate with the memory and to execute the set of instructions, wherein the set of instructions, when executed by the processor, cause the processor to:
obtain patient data about a patient being monitored by the patient monitoring system;
obtain previous patient data;
determine a difference between the patient data and the previous patient data; and
send a message to a user display associated with the patient monitoring system, to cause the user display to adapt content displayed on the user display, based on the determined difference.

2. A patient monitoring system as in claim 1 wherein the processor is further caused to:
select one or more previous patients from a plurality of previous patients; and
wherein the processor is configured to obtain the previous patient data about the selected one or more previous patients.

3. A patient monitoring system as in claim 2 wherein the processor is further configured to determine a current clinical user of the patient monitoring system and
wherein the processor is configured to select one or more previous patients that were treated by i) the current clinical user, ii) a team of clinicians associated with the current clinical user, or iii) a medical facility associated with the current clinical user.

4. A patient monitoring system as in claim 3 wherein the processor being caused to send the message to the user display comprises the processor being caused to send the message to the user display to cause the user display to highlight, to the current clinical user, the difference between the patient and the one or more previous patients.

5. A patient monitoring system as in any one of claims 2 to 4 wherein the processor is configured to select one or more previous patients that have at least one characteristic in common with the patient.

6. A patient monitoring system as in claim 5 wherein the at least one characteristic comprises:
at least one physical characteristic in common with the patient;
at least one symptom in common with the patient;
at least one treatment received in common with the patient; and/or
at least one treatment outcome in common with the patient.

7. A patient monitoring system as in any one of claims 2 to 6 wherein the processor is configured to select one or more previous patients having a similarity metric that satisfies a predetermined criteria.

8. A patient monitoring system as in any one of claims 2 to 6 wherein the processor is caused to:
perform a clustering process on the patient and the plurality of previous patients; and
select the one or more previous patients from a cluster comprising the patient being monitored by the patient monitoring system.

9. A patient monitoring system as in claim 8 wherein the processor is caused to re-perform the clustering process upon a change in the patient data; and
send a second message to the user display to cause the user display to provide a notification if the patient moves into a different cluster.

10. A patient monitoring system as in any one of claims 2 to 6 wherein the processor is further configured to select the one or more previous patients from the plurality of previous patients such that the determined difference between the selected one or more patients and the patient satisfies a predefined criteria.

11. A patient monitoring system as in claim 1 wherein:
the patient data comprises current data about the patient being monitored by the patient monitoring system,
the previous patient data comprises previous data about the patient being monitored by the patient monitoring system; and
wherein the processor is caused to send the message to the user display to cause the user display to indicate differences between the current data and the previous data of the patient.

12. A patient monitoring system as in any one of the preceding claims wherein the processor is caused to send the message to the user display to cause the user display to:
indicate the difference between the patient data and the previous patient data; or
order content displayed on the display so as to highlight the difference.

13. A patient monitoring system as in any one of the preceding claims wherein the patient data and the previous patient data comprise:
physiological measurements;
treatment plans; or
diagnostic data.

14. A method performed by a patient monitoring system, the method comprising:
obtaining (202) patient data about a patient being monitored by the patient monitoring system;
obtaining (204) previous patient data;
determining (206) a difference between the patient data and the previous patient data; and
sending (208) a message to a user display associated with the patient monitoring system, to cause the user display to adapt content displayed on the user display, based on the determined difference.

15. A computer program product comprising a computer readable medium, the computer readable medium having computer readable code embodied therein, the computer readable code being configured such that, on execution by a suitable computer or processor, the computer or processor is caused to perform the method as claimed in claim 14.
